(19) Europäisches Patentamt
European Patent Office
Office européen des brevets

(11) Publication number: **0 524 477 A2**

# (12) EUROPEAN PATENT APPLICATION

(21) Application number: **92111458.3**

(22) Date of filing: **07.07.92**

(51) Int. Cl.[5]: **C08F 20/38**, G02B 1/04, C07C 323/12

(30) Priority: **22.07.91 JP 181132/91**
**14.11.91 JP 298815/91**

(43) Date of publication of application:
**27.01.93 Bulletin 93/04**

(84) Designated Contracting States:
**DE FR GB**

(71) Applicant: **NIPPON SHEET GLASS CO., LTD.**
**5-11, 3-Chome Doshomachi**
**Chuo-ku Osaka(JP)**

(72) Inventor: **Kayanoki, Hisayuki, c/o Nippon Sheet Glass Co.,Ltd**
**3-5-11, Doshomachi, Chuo-ku**
**Osaka(JP)**
Inventor: **Ishizuka, Satoshi, c/o Nippon Sheet Glass Co.,Ltd**
**3-5-11, Doshomachi, Chuo-ku**
**Osaka(JP)**
Inventor: **Takigawa, Akio, c/o Nippon Sheet Glass Co.,Ltd**
**3-5-11, Doshomachi, Chuo-ku**
**Osaka(JP)**

(74) Representative: **Kraus, Walter, Dr. et al**
**Patentanwälte Kraus, Weisert & Partner**
**Thomas-Wimmer-Ring 15**
**W-8000 München 22(DE)**

## (54) Resin composition having high refractive index properties.

(57) An easily moldable resin composition having high refractive index properties, which gives a product having a low specific gravity and excellent heat resistance and processability. The resin composition comprises a polymer obtained by polymerizing an acrylate or methacrylate monomer (a) of the formula (1),

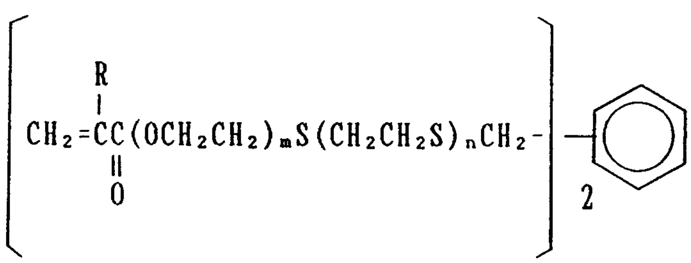

(1)

wherein R is hydrogen or methyl,n is an integer of 2 or 3 when m is 0 or n is an integer of 0, 1, 2 or 3 when m is an integer of 1 or 2
or a mixture of the monomer (a) and other monomer (b) copolymerizable with the monomer (a).

EP 0 524 477 A2

## FIELD OF THE INVENTION

The present invention relates to a resin composition having high refractive index properties, and particularly to a resin composition having high refractive index properties, which gives a product having excellent transparency, a low specific gravity, excellent heat resistance and excellent processability and which has excellent moldability.

## PRIOR ART OF THE INVENTION

A variety of synthetic resins as substitutes for inorganic glass have been studied, proposed and put to practical use. For example, diethylene glycol bisallylcarbonate is among them. Since, however, the refractive index of a product from diethylene glycol bisallylcarbonate is as low as 1.50, various resins have been developed recently, and put to practical use to obtain a product having a high refractive index of nearly 1.60. For example, practically used are a resin having an aromatic ring, a halogen or a sulfur atom in the molecule and a resin having a urethanethio bond, obtained by a reaction between an isocyanate compound and polythiol. Further, a compound containing particularly many sulfur atoms has been proposed as a resin which gives a product having a high refractive index of 1.60 or more.

However, the above conventional resins do not always satisfy all the required properties. That is, a resin containing a halogen to increase the refractive index has a specific gravity of 1.4 or more, or it is heavy. A resin obtained by a urethane reaction is inferior in moldability when cast-molded. The production of a compound having many sulfur atoms often requires the use of special starting materials or multi-stage reactions.

The present inventors have made a diligent study to overcome the above problems of these conventional resins, and arrived at the present invention.

## SUMMARY OF THE INVENTION

It is an object of the present invention to provide a resin composition having high refractive index properties, which has a low specific gravity and gives a product excellent in heat resistance and processability and which is easily moldable.

According to the present invention, there is provided a resin composition having high refractive index properties, which comprises a polymer obtained by polymerizing an acrylate or methacrylate monomer (a) of the formula (1),

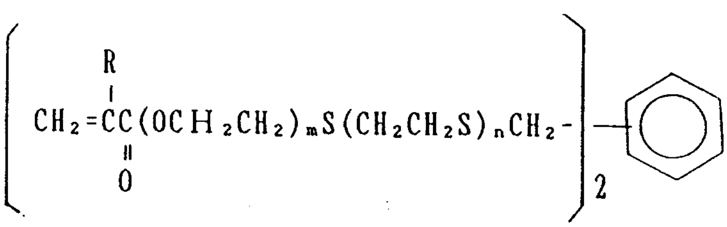

(1)

wherein R is hydrogen or methyl, n is an integer of 2 or 3 when m is 0 and n is an integer of 2 to 3 when m is 0,
or a mixture of the monomer (a) and other monomer (b) copolymerizable with the monomer (a).

## DETAILED DESCRIPTION OF THE INVENTION

The (meth)acrylate monomer (a) of the formula (1) used for the production of the resin composition having high refractive index properties, provided by the present invention, can be produced by the following method.

First, the (meth)acrylate monomer (a) of the formula (1) wherein, $m = 0$, $n = 2$ or 3 can be produced by the following method.

That is, first, $\alpha,\alpha'$-dichloroxylene and a compound of the formula (2),

$H(SCH_2CH_2)_nSH$ (2)

wherein n is an integer of 2 or 3, are allowed to react in an alkaline aqueous solution to synthesize a compound of the formula (3).

$\{HS(CH_2CH_2S)_nCH_2\}_2$-Ph (3)

wherein Ph is phenyl and n is an integer of 2 or 3,

Then, the compound of the formula (3) is converted to a (meth)acrylate to obtain the (meth)acrylate of the formula (1).

The synthesis of the reaction can be carried out by a conventional method. For example, either the compound of the formula (3) is allowed to react with (meth)acrylic acid chloride in toluene in the presence of a polymerization inhibitor and a tertiary amine, or the compound of the formula (3) and (meth)acrylic acid are esterified in toluene in the presence of a polymerization inhibitor and a tertiary amine.

Second, the (meth)acrylate monomer (a) of the formula (1) wherein m = 1 or 2, n = 2 or 3 can be produced by the following method.

That is, 1 mol or 2 mol of ethylene oxide is allowed to bond to each of the two SH groups of the compound of the formula (3), and further the resultant product is converted to a (meth)acrylate by a conventional known method to obtain the (meth)acrylate of the formula (1).

Third, the (meth)acrylate monomer (a) of the formula (1) wherein m = 1 or 2, n = 0 can be produced by the following method.

That is, first, $\alpha,\alpha'$-dichloroxylene and 2-mercaptoethanol are allowed to react in an alkaline aqueous solution to synthesize a compound of the formula (4).

$\{HOCH_2CH_2SCH_2\}_2$-Ph (4)

Then, 1 mol of ethylene oxide may be allowed to bond to each of the two OH groups of the compound of the formula (4), and further the resultant product is converted to a (meth)acrylate by a conventional known method to obtain the (meth)acrylate of the formula (1).

Last, the (meth)acrylate monomer (a) of the formula (1) wherein m = 1 or 2, n = 1 can be produced by the following method.

That is, the compound having SH groups substituted by two OH groups of the compound of the formula (4) is synthesized by a conventional method. Then, 1 mol or 2 mol of ethylene oxide may be allowed to bond to each of the two SH groups of the compound, and further the resultant product is converted to a (meth)acrylate by a conventional known method to obtain the (meth)acrylate of the formula (1).

In the present invention, the above (meth)acrylate monomer (a) alone may be polymerized, and further, a mixture of the monomer (a) with other monomer (b) copolymerizable with the monomer (a) may be polymerized to improve the monomer properties and the polymer properties. However, when the amount of the monomer (b) is too large, the so-obtained resin shows poor moldability and a product obtained from such a resin shows a decrease in some of transparency, surface smoothness, surface hardness, heat resistance, processability, impact resistance, low specific gravity and high refractive index. Therefore, the amount of the (meth)acrylate of the formula (1) in the mixture is preferably at least 20 % by weight based on the total amount of the mixture, more preferably at least 40 % by weight based on the total amount of the mixture.

The above monomer (b) can be selected from compounds having at least one radical-polymerizable functional group including a carbon-carbon double bond in the molecule. Specific examples of the monomer (b) includes maleimides such as N-phenylmaleimide, N-(2-chlorophenyl) maleimide and N-(2,6-diethylphenyl)maleimide; aromatic vinyl compounds such as styrene, divinylbenzene and 4-chlorostyrene; (meth)-acrylates having one functional group such as phenyl methacrylate, phenoxyethyl acrylate, benzyl methacrylate and dicyclopentenyl acrylate; (meth)acrylates having an aromatic ring and two functional groups such as 2,2'-bis(4-acryloxyethoxyphenyl)propane, 2,2'-bis(4-methacryloxyethoxyphenyl)propane, 2,2'-bis(4-methacryloxydiethoxyphenyl)propane, 2,2'-bis(4-methacryloxy-3,5-dibromophenyl)propane, 2,2'-bis(4-methacryloxyethoxy-3,5-dibromophenyl)propane, 2,2'-bis(4-acryloxyphenyl)sulfone, 2,2'-bis(4-acryloxyethoxyphenyl)sulfone, 2,2'-bis(4-acryloxyethoxy-3,5-dibronomophenyl)sulfone, bis(4-acryloxyphenyl)sulfide, bis(4-methacryloxyphenyl)sulfide and bis(4-methacryloxyethoxy-3,5-dibromophenyl)sulfide; methacrylates as aliphatic alcohol derivatives such as ethylene glycol diacrylate, tetraethylene glycol diacrylate, nonaethylene glycol diacrylate, tetraethylene glycol dimethacrylate, 1,6-hexanediol diacrylate, 1,9-nonanediol diacrylate, neopentyl glycol dimethacrylate and trimethylolpropane trimethacrylate; and monomers having an allyl group such as allyl benzoate, diallyl terephthalate, diallyl isophthalate, triallyl trimellitate, tetraallyl pyromellitate and diallyl diphenate.

The method for polymerization of the resin composition of the present invention is not specially limited, and can be selected from known methods. For example, the above monomer or a mixed solution of the above monomers is cured by heating it in the presence of a polymerization initiator in a mold held with a gasket or a spacer thereby to produce a resin molded article. The resin molded article can be also obtained by photopolymerization or radiation polymerization in place of heating. The polymerization initiator is not specially limited, and can be selected from known initiators. Examples of the polymerization initiator for heat curing include azo compounds such as 2,2'-azobis(2-cyclopropylpropionitrile), 2,2'-azobis(2,4-dimethylvaleronitrile) and dimethyl 2,2'-azobis(2-methylpropionate); peroxyesters such as tert-butylperoxyisobutyrate, tert-butylperoxypivalate, tert-butylperoxy(2-ethylhexanoate) and tert-butylperoxyisopropylcarbonate; peroxydicarbonates such as diisopropylperoxydicarbonate; diacyl peroxides such as benzoyl peroxide; dialkyl peroxides such as dicumyl peroxides; peroxyketals such as 1,1-bis(tert-butylperoxy)-cyclohexane; and other peroxides. For curing the resin composition by ultraviolet light, the initiator can be selected from benzoin isopropyl ether, benzophenone and benzoin isobutyl ether, although the initiator shall not be limited to these. The amount of the polymerization initiator based on the monomer is generally 0.001 to 10 % by weight, preferably 0.01 to 5 % by weight. The kind and concentration of the polymerization initiator are determined after consideration of the composition, reactivity and reaction rate control of a mixed monomer solution.

The resin composition of the present invention preferably contains 0.01 to 3.0 % by weight of an ultraviolet light absorber in order to improve the light resistance and weatherability thereof as required. When the concentration of the ultraviolet light absorber is less than 0.01 % by weight, there is almost no effect on the improvement of the light resistance and weatherability. When it exceeds 3.0 % by weight, a product obtained from the composition undesirably shows unambiguous coloring in yellow and its commercial value is liable to decrease. The ultraviolet light absorber used in the present invention widely includes ultraviolet light absorbers such as benzotriazole-containing, benzophenone-containing, salicylic acid-containing and cyanoacrylate-containing absorbers and ultraviolet light stabilizers such as hindered amine-containing, Ni complex-containing and benzoate-containing stabilizers.

Further, the resin composition of the present invention may contain additives such as a mold releasing agent, an antioxidant, an antistatic agent and a variety of stabilizers and bluing agents. A molded article obtained from the resin composition of the present invention may be surface-coated in order to improve the optical and mechanical stability.

The resin composition of the present invention is remarkably superior as a material for optical materials, since it gives a product which is excellent in transparency, surface smoothness, surface hardness, heat resistance, processability and impact resistance and has a specific gravity of 1.39 or less and a higher refractive index than a diethylene glycol bisallylcarbonate resin (CR-39, refractive index about 1.50) or a refractive index of 1.60 or more. Further, the resin composition of the present invention can be molded by a simple molding method and has excellent moldability.

The resin composition of the present invention can be used as a material for the production of lenses such as an ophthalmic lens and camera lens; prisms; disks such as a video disk; mirrors such as a concave mirror and a polygon, optical materials such as an optical fiber, and as a high-refractive-index coating agent.

Examples of the present invention will be described hereinafter. However, the present invention shall not be limited thereto. In Examples, the properties of samples were tested by the following methods.

Moldability: In cast-molding, a composition which gave a product exactly as defined by a mold was taken as being excellent (○), a composition which slightly caused peeling, etc., was taken as being nearly good (△), and a composition which extraordinarily caused peeling, etc., was taken as being inferior (X).

Heat resistance: Sample lenses were allowed to stand in a hot air dryer at 120°C for 1 hour, and then taken out. A sample, on the surface of which no distortion of a reflected image was visually observed, was taken as (○), a sample, on the surface of which slight distortion of a reflected image was visually observed, was taken as (△), and a sample, on the surface of which a sizable distortion of a reflected image was visually observed, was taken as (X). Further, sample lenses were allowed to stand in a hot air dryer at 130°C for 1 hour, and taken out. In this case, a sample, on the surface of which no distortion of a reflected image was visually observed, was taken as (◎).

Processability: Sample lenses were polished with a grinder for processing an ophthalmic lens. A sample having an excellent polished surface was taken as being excellent (○), a sample having a nearly good polished surface was taken as being nearly good (△), and a sample having a poor surface was taken as being inferior (X).

Appearance: A colorless transparent sample was taken as (○), a sample slightly colored in yellow was taken as (△), and a sample considerably colored in yellow was taken as (X).

Refractive index and Abbe's number: Measured with an Abbe's refractometer.

Example 1

17.5 Grams (0.1 mol) of α,α'-dichloro-m-xylene and 32.4 g (0.21 mol) of mercaptoethyl sulfide were allowed to react in an alkaline aqueous solution to give 28.7 g of a compound (A) of the formula (5).

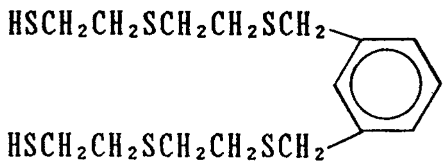

(5)

Then, 20.5 g (0.05 mol) of the compound (A) was allowed to react with 10.9 g (0.12 mol) of acrylic acid chloride in 200 ml of toluene in the presence of 100 mg of a polymerization inhibitor and 8.4 g (0.11 mol) of pyridine to give 18.2 g of a compound (B) of the formula (6).

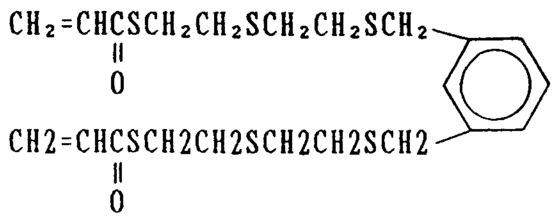

(6)

A solution prepared by adding 2 parts of tert-butylperoxy(2-ethylhexanoate) as a polymerization initiator to 100 parts of the compound (B) was cast into a mold formed of glass mold elements and plastic gaskets, and the solution temperature was elevated from 50 ° C to 110 ° C by taking 20 hours for polymerization. The so-obtained lens was colorless and transparent and had a refractive index of 1.66. Table 1 shows the moldability, heat resistance, processability, appearance, Abbe's number and specific gravity.

Example 2

A compound (B-2) was obtained in the same manner as in Example 1 except that the acrylic acid chloride was replaced with methacrylic acid chloride. This compound is represented by the formula (1) in which R is hydrogen, m is 0 and n is 3. Then, this compound was polymerized in the same manner as in Example 1 to give a colorless transparent lens. Table 1 shows the results.

Example 3

A compound (B-3) was obtained in the same manner as in Example 1 except that α,α'-dichloro-m-xylene was replaced with α,α'-dichloro-p-xylene. This compound is represented by the formula (1) in which R is hydrogen, m is 0 and n is 3. Then, this compound was polymerized in the same manner as in Example 1 to give a colorless transparent lens. Table 1 shows the results.

Example 4

A solution prepared by adding 2 parts of tert-butylperoxy(2-ethylhexanoate) as a polymerization initiator to a mixture of 50 parts of the compound (B) and 50 parts of benzyl methacrylate was polymerized in the same manner as in Example 1 to give a colorless transparent lens. Table 1 shows the results.

Examples 5~7

Colorless transparent lenses were obtained from the compound (B), the compound (B-2), the compound (B-3) and other polymerizable compounds such as divinyl benzene, diallyl diphenate and N-(2,6-diethylphenylmaleimide). Table 1 shows the results.

Table 1

| Example | Composition and compositional ratio | (wt.%) | Mold-ability | Heat resistance |
|---|---|---|---|---|
| 1 | m-XDS-A | (100) | ○ | ○ |
| 2 | m-XDS-MA | (100) | ○ | ○ |
| 3 | p-XDS-A | (100) | ○ | ○ |
| 4 | m-XDS-A/BzMA | (50/50) | ○ | ○ |
| 5 | m-XDS-A/DVB | (60/40) | ○ | ○ |
| 6 | m-XDS-A/DPA | (50/50) | ○ | ○ |
| 7 | m-XDS-A/EPMI | (80/20) | ○ | ○ |

| Example | Process-ability | Appea-rance | Refractive index | Abbe's number | Specific gravity |
|---|---|---|---|---|---|
| 1 | ○ | ○ | 1.66 | 34 | 1.31 |
| 2 | ○ | ○ | 1.65 | 34 | 1.31 |
| 3 | ○ | ○ | 1.66 | 34 | 1.31 |
| 4 | ○ | ○ | 1.61 | 35 | 1.26 |
| 5 | ○ | ○ | 1.64 | 33 | 1.26 |
| 6 | ○ | ○ | 1.63 | 33 | 1.28 |
| 7 | ○ | ○ | 1.61 | 34 | 1.30 |

Abbreviations for compounds:

m-XDS-A: Acrylate from meta-form as a starting material (compound (B)).

m-XDS-MA: Methacrylate from meta-form as a starting material (compound (B-2))

p-XDS-A: Acrylate from para-form as a starting material (compound (B-3))

BzMA:. Benzyl methacrylate

DVB: Divinyl Benzen

DPA: Diallyl diphenate

EPMI: N-(2,6-diethylphenylmaleimide)

Example 8

A solution prepared by adding 2 parts of tert-butylperoxy-2-ethylhexanoate as a polymerization initiator to 100 parts of an acrylate compound (C) of the following formula (7) obtained from α,α'-dichloro-m-xylene, i.e., a compound of the formula (1) wherein R is hydrogen, m is 1 and n is 1, was cast into a mold formed of glass mold elements and plastic gaskets, and the solution temperature was elevated from 50 ° C to 110 ° C by taking 20 hours for polymerization. The so-obtained lens was colorless and transparent and had a refractive index of 1.63. Table 2 shows the moldability, heat resistance, processability, appearance, Abbe's number and specific gravity.

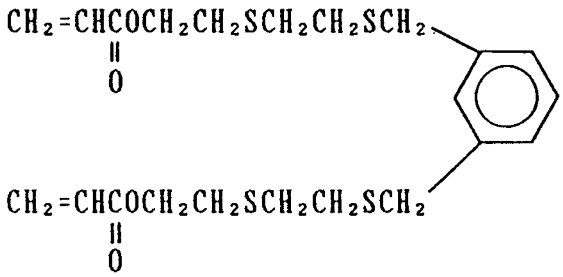

(7)

Example 9

A transparent colorless lens was obtained in the same manner as in Example 8 except that the acrylate compound was replaced with an acrylate compound of the formula (1) in which R was hydrogen, m was 1 and n was 1, obtained from α,α'-dichloro-p-xylene. Table 2 shows the results.

Example 10

A transparent colorless lens was obtained in the same manner as in Example 8 except that the acrylate compound was replaced with an acrylate compound of the formula (1) in which R was hydrogen, m was 1 and n was 2, obtained from α,α'-dichloro-m-xylene. Table 2 shows the results.

Example 11

A transparent colorless lens was obtained in the same manner as in Example 8 except that the acrylate compound was replaced with an acrylate compound of the formula (1) in which R was hydrogen, m was 1 and n was 0, obtained from α,α'-dichloro-m-xylene. Table 2 shows the results.

Example 12

A transparent colorless lens was obtained in the same manner as in Example 8 except that the acrylate compound was replaced with an acrylate compound of the formula (1) in which R was hydrogen, m was 1 and n was 3, obtained from α,α'-dichloro-m-xylene. Table 2 shows the results.

Example 13

A solution prepared by adding 2 parts of tert-butylperoxy-(2-ethylhexanoate) as a polymerization initiator

to a mixture of 50 parts of the compound (C) and 50 parts of benzyl methacrylate was polymerized in the same manner as in Example 8 to give a colorless transparent lens. Table 2 shows the results. Due to the addition of the above benzyl methacrylate, the viscosity of the solution decreased, which decrease made it possible to cast the solution into the mold rapidly.

Examples 14~16

Colorless transparent lenses were obtained from the compound (C) and other polymerizable compounds such as divinyl benzene, diallyl diphenate and N-(2,6-diethylphenyl)maleimide. Table 2 shows the results. Due to the addition of the above benzyl methacrylate in Example 14, the viscosity of the solution decreased, which decrease made it possible to cast the solution into the mold rapidly.

Table 2

| Example | Composition and compositional ratio | (wt.%) | Mold-ability | Heat resistance |
|---|---|---|---|---|
| 8 | m-X1S-A | (100) | ○ | ○ |
| 9 | p-X1S-MA | (100) | ○ | ○ |
| 10 | m-X2S-A | (100) | ○ | ○ |
| 11 | m-X0S-A | (100) | ○ | ○ |
| 12 | m-X3S-A | (100) | ○ | ○ |
| 13 | m-X1S-A/BzMA | (50/50) | ○ | ○ |
| 14 | m-X1S-A/DVB | (60/40) | ○ | ○ |
| 15 | m-X1S-A/DPA | (50/50) | ○ | ○ |
| 16 | m-X1S-A/EPMI | (80/20) | ○ | ◎ |

| Example | Process-ability | Appea-rance | Refractive index | Abbe's number | Specific gravity |
|---|---|---|---|---|---|
| 8 | ○ | ○ | 1.63 | 34 | 1.31 |
| 9 | ○ | ○ | 1.63 | 34 | 1.31 |
| 10 | ○ | ○ | 1.64 | 34 | 1.31 |
| 11 | ○ | ○ | 1.62 | 34 | 1.31 |
| 12 | ○ | ○ | 1.64 | 34 | 1.31 |
| 13 | ○ | ○ | 1.60 | 35 | 1.26 |
| 14 | ○ | ○ | 1.62 | 33 | 1.26 |
| 15 | ○ | ○ | 1.61 | 33 | 1.28 |
| 16 | ○ | ○ | 1.62 | 34 | 1.30 |

Abbreviations for compounds

m-X1S-A: Acrylate form meta-form as a starting material, compound of formula (1) wherein R = hydrogen, m = 1 and n = 1

p-X1S-A: Acrylate form para-form as a starting material, compound of formula (1) wherein R = hydrogen, m = 1 and n = 1

m-X2S-A: Acrylate form meta-form as a starting material, compound of formula (1) wherein R = hydrogen, m = 1 and n = 2

m-X0S-A: Acrylate form meta-form as a starting material, compound of formula (1) wherein R = hydrogen, m = 1 and n = 0

m-X3S-A: Acrylate form meta-form as a starting material, compound of formula (1) wherein R = hydrogen, m = 1 and n = 3

BzMA: Benzyl methacrylate

DVB: Divinyl Benzen

DPA: Diallyl diphenate

DPMI: N-(2,6-diethylphenyl)maleimide

Example 17

A compound of the formula (3) in which n was 1 was obtained from $\alpha,\alpha'$-dichloro-m-xylene as a starting material, and 1 mol or 2 mol of ethylene oxide was allowed to bond to each of the SH groups thereof to give a mixture of the addition products, a precursor of an intended compound of the formula (1) in which R was hydrogen, m was 1.3, and n was 1. Then, the mixture was allowed to react with acrylic acid to synthesize the intended compound. 2 Parts of tert-butylperoxy-2-ethylhexanoate as a polymerization initiator was added to 100 parts of the intended compound, and the resultant solution was cast into a mold formed of glass mold elements and plastic gaskets. The solution temperature was elevated from 50 °C to 110 °C by taking 20 hours for polymerization. The so-obtained lens was colorless and transparent and had a refractive index of 1.63, an Abbe's number of 34 and a specific gravity of 1.31. The moldability, heat resistance, processability and appearance were excellent.

**Claims**

**1.** A resin composition having high refractive index properties, which, comprises a polymer obtained by polymerizing an acrylate or methacrylate monomer (a) of the formula (1),

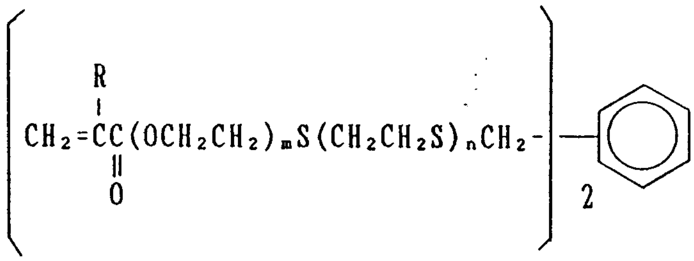

(1)

wherein R is hydrogen or methyl, n is an integer of 2 or 3 when m is 0 or n is an integer of 0, 1, 2 or 3 when m is an integer of 1 or 2
or a mixture of the monomer (a) and other monomer (b) copolymerizable with the monomer (a).

**2.** A resin composition according to claim 1, wherein at least 20 % by weight of the monomer (a) is contained in the mixture.

**3.** A resin composition according to claim 1, wherein the monomer (b) is a compound having at least one radical-polymerizable functional group including a carbon-carbon double bond in the molecule.

**4.** A resin composition according to claim 1, wherein the monomer (b) is selected from maleimides, vinyl compounds, acrylates or methacrylates having one functional group, acrylates or methacrylates having an aromatic ring and two functional groups, methacrylates as aliphatic alcohol derivatives and monomers having an allyl group.